# EUROPEAN PATENT SPECIFICATION

(11) **EP 0 881 300 B1**
(45) Date of publication and mention of the grant of the patent: **17.01.2001**
(21) Application number: 98304247.4
(22) Date of filing: 29.05.1998
(51) Int. Cl.: C12Q 1/26, C12Q 1/44, C12Q 1/533, G01N 33/50

(54) **Method for identifying compounds for inhibition of neoplastic lesions**
Verfahren zur Identifizierung von Verbindungen zur Hemmung von neoplastischen Verletzungen
Méthode pour l'identification de composés pour l'inhibition des lésions néoplastiques

(30) Priority: 30.05.1997 US 866027; 24.03.1998 US 46739
(43) Date of publication of application: 02.12.1998
(60) Divisional application: 00112020.3
(73) Proprietor: Cell Pathways, Inc., Horsham, Pennsylvania 19044 (US)
(72) Inventor: Pamukcu, Rifat, Spring House, PA 19477 (US); Piazza, Gary A., Doylestown, PA 18901 (US); Thompson, W. Joseph, Mobile, AL 36608 (US)
(74) Representative: Baverstock, Michael George Douglas

(56) References cited:
- WO-A-95/18969
- WO-A-95/26743
- J. A. MITCHELL ET AL.: "Selectivity of nonsteroidal antiinflammatory drugs as inhibitors of constitutive and inducible cyclooxygenase" PROCEEDINGS OF THE NATIONAL ACADEMY OF SCIENCES OF USA., vol. 90, December 1994, pages 11693-11697, XP002084859 WASHINGTON US
- CHEMICAL ABSTRACTS, vol. 106, no. 11, 16 March 1987 Columbus, Ohio, US; abstract no. 78377, J. D. GAFFEN ET AL.: "Increased killing of malignant cells by giving indomethacin with methotrexate." page 30; column 1; XP002084860 & PROG. LIPID RES., vol. 25(Essent. Fatty Acids, Prostaglandins Luekotrien), 1986, pages 543-545,

## Description

### BACKGROUND OF THE INVENTION

This invention provides a method for identifying compounds potentially useful for the treatment and prevention of pre-cancerous and cancerous lesions in mammals.

Familial adenomatous polyposis ("FAP") is an inherited disease where the victim's colon contains many polyps or adenomas virtually uncountable in most instances. Because such patients develop so many polyps or adenomas each of which has a significant risk of developing into a cancer -- the typical treatment is surgical removal of the colon. In about 1983, Waddell discovered that the nonsteroidal anti-inflammatory drug ("NSAID") sulindac would cause colonic polyps (a type of pre-cancerous lesion) to regress and prevent their recurrence when that drug was administered to patients with FAP. Waddell's experience with sulindac in FAP patients was confirmed in several subsequent studies. Unfortunately, since sulindac and other NSAIDS aggravate the digestive tract (not to mention side effects involving kidney and interference with normal blood clotting) of patients to whom it has been chronically administered, it is not a practical treatment for FAP or any other cancer or precancerous indication (i.e., neoplasia) requiring long-term administration.

Waddell originally hypothesized that the mechanism of action of sulindac on colonic polyps involved the inhibition of the synthesis of prostaglandin (PG). (Waddell, W.R. et al., "Sulindac for Polyposis of the Colon," *Journal of Surgical Oncology,* 24:83-87, 1983). Prostaglandin ("PG") synthesis inhibition results from the inhibition of cyclooxygenase (COX) caused by NSAIDs. A common benefit of NSAIDs is the reduction of inflammation, which is known to be caused by the reduction of PG levels. Since NSAIDs are known to inhibit COX, which inhibits PG synthesis, it is widely believed that the regression of colonic polyps is attributed to this property. In fact, notwithstanding recent discoveries to the contrary, it has become conventional wisdom that administration of an inhibitor of PG synthesis (e.g., an NSAID) to a patient with FAP or other precancerous or cancerous lesion will result in the regression of the lesion due to a reduction of PG levels. In fact, researchers specifically ruled out phosphodiesterase as a target (see J.D. Gaffen et al.; Increased killing of malignant cells by giving indomethacin with methotrexate, Prog. Lipid Res., 25, pp. 543-545, 1986).

Recent discoveries, however, are leading scientists in a completely different direction - that it is not necessary to inhibit COX to treat neoplasia patients successfully. Pamukcu et al., in U.S. Patent No. 5,401,774, disclosed that sulfonyl compounds, that were previously reported to be inactive as PG synthesis inhibitors (and therefore not an NSAID or an anti-inflammatory compound) unexpectedly inhibited the growth of a variety of neoplastic cells, including colon polyp cells. These sulfonyl derivatives have proven effective in rat models of colon carcinogenesis, and one variant (now referred to as exisulind) has proven effective in preliminary human clinical trials with FAP patients.

The importance of this discovery - and the de-linking of anti-neoplasitic activity and COX inhibition - cannot be overstated. If those two phenomena were related, there would be little hope for a safe NSAID therapy for FAP patients because the side effects of NSAIDs, such as gastric irritation, are also caused by COX inhibition. Prostaglandins play a protective function in the lining of the stomach. When NSAIDs are administered, COX is inhibited and PG levels are reduced: gastric irritation is a common result. Those side effects may not manifest themselves in short-term (acute) NSAID therapy. However, during long-term (chronic) NSAID therapy, gastric irritation, bleeding and ulceration are very common. In significant numbers of cases, NSAID therapy must be stopped due to the severity of those side effects and other potentially lethal side effects. Furthermore, the severity of such side effects increases with age, probably because natural PG levels in gastric mucosa falls with age. Thus, useful compounds for treating neoplastic lesions should desirably inhibit neoplastic cell growth, but should not inhibit COX.

Conventional methods for screening compounds may be used to find improved compounds that inhibit neoplastic cell growth. Under this scenario, drugs may be screened using *in vitro* models. But conventional *in vitro* screening methods could pass many compounds that later are shown to be ineffective in animal models because of a number of unanticipated problems, one of which may be that the *in vitro* screen is not predictive of efficacy. Animal model studies are time consuming and expensive. Therefore, a more precise *in vitro* screening method that provides predictive information for treating neoplasia is needed to screen compounds prior to human testing. Knowledge of a specific target for inhibiting human cancer would allow for greater precision and efficiency whereby highly effective and safe compounds can be identified prior to animal testing.

Presently, rational drug discovery methods are being applied in the pharmaceutical industry to improve methods for identifying clinically useful compounds. Typically, rational drug discovery methods relate to a "lock and key" concept whereby structural relationships between a therapeutic target molecule (lock) and pharmaceutical compounds (key) are defined. Such methods are greatly enhanced by specialty computer software that accesses databases of compounds to identify likely geometric fits with the target molecule. Unfortunately, to use these systems, one has to have insight to the target molecule (lock). The target may be an enzyme, a protein, a membrane or nuclear receptor, or a nucleic acid sequence, for example.

In complex diseases, such as neoplasia, scientists have identified a number of potential targets. However, many of the drugs available for the treatment of neoplasia are non-specific and toxic to normal tissues, and are not indicated for precancer and used only when neoplastic cells progress to cancer. Greater understanding of the mechanisms involved in cancer may lead scientists on the path towards designing more specific antineoplastic drugs -- drugs that can safely be administered earlier in the disease process.

### SUMMARY OF THE INVENTION

This invention relates to a novel *in vitro* method for screening test compounds for their ability to treat and prevent neoplasia, especially pre-cancerous lesions, safely. In particular, the present invention provides a method for identifying test compounds that can be used to treat and prevent neoplasia, including precancerous lesions, with minimal side effects associated with COX inhibition and other non-specific interactions.

In one embodiment of this invention, therefore, the screening method involves determining the COX inhibition activity of a test compound. Because the inventors have discovered a relationship between inhibition of cancer and inhibition of phosphodiesterase Type-5 isoenzyme ("PDE5"), this invention includes determining the PDE5 inhibition activity of the compound. Preferably, the screening method of this invention further includes determining whether the compounds inhibit the growth of tumor cells in a cell culture.

In an alternate embodiment, the screening method of this invention involves determining the COX inhibition activity of the compound, determining the PDE5 inhibition activity of the compound and determining whether the compound induces apoptosis in tumor cells.

By screening compounds in this fashion, potentially beneficial and improved compounds can be identified more rapidly and with greater precision than possible in the past. Further benefits will be apparent from the detailed description that follows.

### BRIEF DESCRIPTION OF THE DRAWINGS

Figure 1 illustrates the effect of the sulfide derivative of sulindac and the sulfone derivative of sulindac (a.k.a. exisulind) on purified cyclooxygenase activity.

Figure 2 illustrates the effects of test compounds B and E on COX inhibition.

Figure 3 illustrates the inhibitory effects of sulindac sulfide and exisulind on PDE-4 and PDE5 purified from cultured tumor cells.

Figure 4 illustrates the effects of sulindac sulfide on cyclic nucleotide levels in HT-29 cells.

Figure 5 illustrates the phosphodiesterase inhibitory activity of compound B.

Figure 6 illlustrates the phosphodiesterase inhibitory activity of compound E.

Figure 7 illustrates the effects of sulindac sulfide and exisulind on apoptosis and necrosis of HT-29 cells.

Figure 8 illustrates the effects of sulindac sulfide and exisulind on HT-29 cell growth inhibition and apoptosis induction as determined by DNA fragmentation.

Figure 9 illustrates the apoptosis inducing properties of compound E.

Figure 10 illustrates the apoptosis inducing properties of compound B.

Figure 11 illustrates the effects of sulindac sulfide and exisulind on tumor cell growth.

Figure 12 illustrates the growth inhibitory and apoptosis-inducing activity of sulindac sulfide and control (DMSO).

Figure 13 illustrates the growth inhibitory activity of compound E.

Figure 14 illustrates the inhibition of pre-malignant, neoplastic lesions in mouse mammary gland organ culture by sulindac metabolites.

### DETAILED DESCRIPTION OF THE PREFERRED EMBODIMENTS

The method of this invention is useful to identify compounds that can be used to treat or prevent neoplasms, and which are not characterized by the serious side effects of conventional NSAIDs.

Cancer and precancer may be thought of as diseases that involve unregulated cell growth. Cell growth involves a number of different factors. One factor is how rapidly cells proliferate, and another involves how rapidly cells die. Cells can die either by necrosis or apoptosis depending on the type of environmental stimuli. Cell differentiation is yet another factor that influences tumor growth kinetics. Resolving which of the many aspects of cell growth is affected by a test compound is important to the discovery of a relevant target for pharmaceutical therapy. Screening assays based on this selectivity can be combined with tests to determine which compounds having growth inhibiting activity.

This invention is the product of several important discoveries. First, the present inventors discovered that desirable inhibitors of tumor cell growth induce premature death of cancer cells by apoptosis (see, Piazza, G.A., et al., *Cancer Research,* 55(14), 3110-16, 1995). Second, the present inventors unexpectedly discovered that compounds that selectively induce apoptosis without substantial COX inhibition also inhibit phosphodiesterase ("PDE"). In particular, and contrary to leading scientific studies, desirable compounds for treating neoplastic lesions selectively inhibit Type 5 isoenzyme form of phosphodiesterase ("PDE5") (EC 3.1.4.17). PDE5 is one of at least seven isoenzymes of phosphodiesterase. PDE5 is unique in that it selectively degrades cyclic GMP, while the other types of PDE are either non-selective or degrade cyclic AMP. Preferably, desirable compounds do not substantially inhibit other phosphodiesterase types.

A preferred embodiment of the present invention involves determining the cyclooxygenase inhibition activity of a given compound, and determining the PDE5 inhibition activity of the compound. The test compounds are scored for their probable ability to treat neoplastic lesions either directly by assessing their activities against specific cutoff values or indirectly by comparing their activities against known compounds useful for treating neoplastic lesions. A standard compound that is known to be effective for treating neoplastic lesions without causing gastric irritation is 5-fluoro-2-methyl-1-(p-methylsulfonylbenzylidene)-3-indenylacetic acid ("exisulind"). Other useful compounds for comparative purposes include those that are known to inhibit COX, such as indomethacin and the sulfide metabolite of sulindac: 5-fluoro-2-methyl-1-(p-methylsulfinylbenzylidene)-3-indenylacetic acid ("sulindac sulfide"). Other useful compounds for comparative purposes include those that are known to inhibit PDE5, such as 1-(3-chloroanilino)-4-phenyphthalazine ("MY5445").

A test compound is clearly determined to be a promising candidate if it performs better than or comparable to exisulind and does not inhibit COX. In general, desirable compounds are those that inhibit PDE5 and inhibit cell growth and induce apoptosis, but do not inhibit COX at pharmacologically accepted doses.

As used herein, the term "precancerous lesion" includes syndromes represented by abnormal neoplastic, including dysplastic, changes of tissue. Examples include dysplastic growths in colonic, breast, prostate or lung tissues, or conditions such as dysplastic nevus syndrome, a precursor to malignant melanoma of the skin. Examples also include, in addition to dysplastic nevus syndromes, polyposis syndromes, colonic polyps, precancerous lesions of the cervix (i.e., cervical dysplasia), esophagus, lung, prostatic dysplasia, prostatic intraneoplasia, breast and/or skin and related conditions (e.g., actinic keraosis), whether the lesions are clinically identifiable or not.

As used herein, the term "carcinoma" or "cancer" refers to lesions which are cancerous. Examples include malignant melanomas, breast cancer, prostate cancer and colon cancer. As used herein, the terms "neoplasia" and "neoplasms" refer to both cancerous and pre-cancerous lesions.

As used herein, the abbreviation PG represents prostaglandin; PS represents prostaglandin synthetase; PGE₂ represents prostaglandin E₂; PDE represents phosphodiesterase; COX represents cyclooxygenase; RIA represents - radioimmunoassay.

As used herein, "PDE5" refers to that enzyme and any of its isoforms that exhibit cGMP specific hydrolytic enzyme activities and high affinity cGMP binding.

In another aspect, there is a method for treating patients in need of treatment for neoplasia by identifying compounds that exhibit substantial PDE5 inhibitory activity at pharmacologically acceptable doses, and administering one or more of those compounds to a patient in need thereof with neoplasia sensitive to the compound.

### SCREENING PROTOCOLS

The following screening protocols, and alternative protocols, are provided to aid in the understanding of the preferred methods used to screen test compounds to determine their potential to treat or prevent neoplasia, especially pre-cancerous lesions.

### 1. Determining COX Inhibitory Activity

COX inhibition can be determined by either of two methods. One method involves measuring PGE₂ secretion by intact HL-60 cells following exposure to the compound being screened. The other method involves measuring the activity of purified cyclooxygenases (COXs) in the presence of the compound. Both methods involve protocols previously described in the literature.

### 1.A. PGE₂ secretion

Compounds of this can be evaluated to determine whether they inhibited the production of prostaglandin E₂ ("PGE₂"), according to procedures known in the art. For example, PGE₂ secreted from a cell can be measured using an enzyme immunoassay (EIA) kit for PGE₂, such as commercially available from Amersham, Arlington Heights, IL USA. Suitable cells include those which make an abundance of PG, such as HL-60 cells. HL-60 cells are human promyelocytes that are differentiated with DMSO in mature granulocytes. (See, Collins, S.J., Ruscetti, F.W., Gallagher, R.E. and Gallo, R.C., "Normal Functional Characteristics of Cultured Human Promyelocytic Leukemia Cells (HL-60) After Induction of Differentiation By Dimethylsulfoxide", *J. Exp. Med.,* 149:969-974, 1979). These differentiated cells produce PGE₂ after stimulation with a calcium ionophore A23187 (see, Kargman, S., Prasit, P. and Evans, J.F., "Translocation of HL-60 Cell 5-Lipoxygenase", *J. Biol. Chem.,* 266: 23745-23752, 1991). HL-60 are available from the American Type Culture Collection (ATCC:CCL240). They can be grown in a RPMI 1640 medium supplemented with 20% heat-inactivated fetal bovine serum, 50 U/ml penicillin and 50 µg/ml streptomycin in an atmosphere of 5% CO₂ at 37°C. To induce myeloid differentiation, cells are exposed to 1.3% DMSO for 9 days and then washed and resuspended in Dulbecco's phosphate-buffered saline at 3x10⁶ cells/ml.

The differentiated HL-60 cells (3x10⁶ cells/ml) can be incubated for 15 min at 37°C in the presence of the compounds tested at the desired concentration. Cells are then stimulated by A23187 (5x10⁻⁶ M) for 15 min. PGE₂ secreted into the external medium is measured as described above.

### 1.B Purified cyclooxygenases

Two different forms of cyclooxygenase (COX-I and COX-2) have been reported in the literature to regulate prostaglandin synthesis. It is known that COX-2 represents the inducible form of COX while COX-I represents a constitutive form. COX-I activity can be measured using the method described by Mitchell et al. ("Selectivity of Nonsteroidal Anti-inflammatory Drugs as Inhibitors of Constitutive and Inducible Cyclooxygenase," *Proc. Natl. Acad. Sci. USA.,* 90:11693-11697, 1993) using COX-I purified from ram seminal vesicles as described by Boopathy & Balasubramanian, "Purification And Characterization Of Sheep Platelet Cyclooxygenase" (*Biochem. J.*, 239:371-377, 1988). COX-2 activity can be measured using COX-2 purified from sheep placenta as described by Mitchell et al., 1993, *supra.*

The cyclooxygenase inhibitory activity of a drug can be determined by methods known in the art. For example, Boopathy & Balasubramanian, 1988, *supra,* described a procedure in which prostaglandin H synthase 1 (Cayman Chemical, Ann Arbor, Michigan) is incubated at 37°C for 20 min with 100 µM arachidonic acid (Sigma Chemical Co.), cofactors (such as 1.0 mM glutathione, 1.0 mM hydroquinone, 0.625 µM hemoglobin and 1.25 mM CaCl₂ in 100 mM Tri-HCl, pH 7.4) and the drug to be tested. Following incubation, the reaction can be terminated with trichloroacetic acid. Enzymatic activity can then be measured spectrophotometrically at 530 nm after stopping the reaction by adding thiobarbituric acid and malonaldehyde.

Obviously, a compound that exhibits minimal COX-I or COX-2 inhibitory activity in relation to its greater PDES inhibitory activity may not be entirely undesirable.

### 1.C. Analyzing Results

The amount of inhibition is determined by comparing the activity of the cyclooxygenase in the presence and absence of the test compound. Residual or no COX inhibitory activity (i.e., less than about 25%) at a concentration of about 100 µM is indicative that the compound should be evaluated further for usefulness for treating neoplasia. Preferably, the IC₅₀ concentration should be greater than 1000 µM for the compound to be further considered potential use.

### 2. Determining Phosphodiesterase (PDE5) Inhibition Activity

Compounds can be screened for inhibitory effect on phosphodiesterase activity using either the enzyme isolated from any tumor cell line such as HT-29 or SW-480, or recombinant HS-PDE5, for example, or measuring cyclic nucleotide levels in whole cells.

### 2.A. Enzyme Assay

Phosphodiesterase activity can be determined using methods known in the art, such as a method using radioactive ³H cyclic GMP (cGMP)(cyclic 3',5'-guanosine monophosphate) as the substrate for PDE5 enzyme. (Thompson, W.J., Teraski, W.L., Epstein, P.M., Strada, S.J., *Advances in Cyclic Nucleotide Research,* 10:69-92, 1979). In brief, a solution of defined substrate ³H-cGMP specific activity (0.2 µM; 100,000 cpm; containing 40 mM Tris-HCl (pH 8.0), 5 mM MgCl₂ and 1 mg/ml BSA) is mixed with the drug to be tested in a total volume of 400µl. The mixture is incubated at 30°C for 10 minutes with partially purified PDE5 isolated from HT-29 cells. Reactions are terminated, for example, by boiling the reaction mixture for 75 seconds. After cooling on ice, 100 µl of 0.5 mg/ml snake venom (*O. Hannah* venom available from Sigma) is added and incubated for 10 min at 30°C. This reaction is then terminated by the addition of an alcohol, e.g. 1 ml of 100% methanol. Assay samples are applied to a anion chromatography column (1 ml Dowex, from Aldrich) and washed with 1 ml of 100% methanol. The amount of radioactivity in the breakthrough and the wash from the columns in then measured with a scintillation counter. The degree of PDE5 inhibition is determined by calculating the amount of radioactivity in drug-treated reactions and comparing against a control sample (a reaction mixture lacking the tested compound).

### 2.B. Cyclic Nucleotide Measurements

Alternatively, the ability for desirable compounds to inhibit PDE5 is reflected by an increase in cGMP in neoplastic cells exposed to a compound being screened. The amount of PDE5 activity can be determined by assaying for the amount of cyclic GMP in the extract of treated cells using radioimmunoassay (RIA). In this procedure, HT-29 or SW-480 cells are plated and grown to confluency. The test compound is then incubated with the cell culture at a concentration of compound between about 200 µM to about 200 pM. About 24 to 48 hours thereafter, the culture media is removed from the cells, and the cells are solubilized. The reaction is stopped by using 0.2N HCl/50% MeOH. A sample is removed for protein assay. Cyclic GMP is purified from the acid/alcohol extracts of cells using anion-exchange chromatography, such as a Dowex column. The cGMP is dried, acetylated according to published procedures, such as using acetic anhydride in triethylamine, (Steiner, A.L., Parker, C.W., Kipnis, D.M., *J. Biol. Chem.,* 247(4):1106-13, 1971). The acetylated cGMP is quantitated using radioimmunoassay procedures (Harper, J., Brooker, G., *Advances in Nucleotide Research,* 10:1-33, 1979). Iodinated ligands (tyrosine metheyl ester) of derivatized cyclic GMP are incubated with standards or unknowns in the presence of antisera and appropriate buffers. Antiserum may be produced using cyclic nucleotide-haptene directed techniques. The antiserum is from sheep injected with succinyl-cGMP-albumin conjugates and diluted 1/20,000. Dose-interpolation and error analysis from standard curves are applied as described previously (Seibert, A.F., Thompson, W.J., Taylor, A., Wilbourn, W.H., Barnard, J. and Haynes, J., *J. Applied Physiol*., 72:389-395, 1992).

In addition, the culture media may be acidified, frozen (-70°C) and also analyzed for cGMP and cAMP.

In addition to observing increases in content of cGMP caused by desirable test compounds, decreases in content of cAMP have been observed. It has been observed that a particularly desirable compound (i.e. one that selectively induces apoptosis in neoplastic cells, but not substantially in normal cells) follows a time course consistent with PDE5 inhibition as one initial action resulting in an increased cGMP content within minutes. Secondarily, treatment of neoplastic cells with a desirable anti-neoplastic compound leads to decreased cAMP content within 24 hours. The intracellular targets of drug actions are being studied further, but current data supports the concept that both the initial rise in cGMP content followed by the subsequent fall in cAMP content precede apoptosis in neoplastic cells exposed to desirable compounds.

The change in the ratio of the two cyclic nucleotides may be a more accurate tool for evaluating desirable PDE5 inhibition activity of test compounds, rather than measuring only the absolute value of cGMP, only PDE5 inhibition, or only the absolute value of cGMP. In neoplastic cells not treated with anti-neoplastic compounds, the ratio of cGMP content/cAMP content is in the 0.03-0.05 range (i.e., 300-500 fmol/mg protein cGMP content over 6000-8000 fmol/mg protein cAMP content). After exposure to desirable anti-neoplastic compounds, that ratio increases several fold (preferably at least about a three-fold increase) as the result of an initial increase in cyclic GMP and the later decrease in cyclic AMP.

Specifically, it has been observed that particularly desirable compounds achieve an initial increase in cGMP content in treated neoplastic cells to a level of cGMP greater than about 500 fmol/mg protein. In addition, particularly desirable compounds cause the later decrease in cAMP content in treated neoplastic cells to a level of cAMP less than about 4000 fmol/mg protein.

To determine the content of cyclic AMP, radioimmunoassay techniques similar to those described above for cGMP are used. Basically, cyclic nucleotides are purified from acid/alcohol extracts of cells using anion-exchange chromatography, dried, acetylated according to published procedures and quantitated using radioimmunoassay procedures. lodinated ligands of derivatized cyclic AMP and cyclic GMP are incubated with standards or unknowns in the presence of specific antisera and appropriate buffers.

Verification of the cyclic nucleotide content may be obtained by determining the turnover or accumulation of cyclic nucleotides in intact cells. To measure inteact cell cAMP, ³H-adenine prelabeling is used according to published procedures (Whalin M.E., R.L. Garrett Jr., W.J. Thompson, and S.J. Strada, "Correlation of cell-free brain cyclic nucleotide phosphodiesterase activities to cyclic AMP decay in intact brain slices", *Sec. Mess. and Phos. Protein Research,* 12:311-325, 1989). The procedure measures flux of labeled ATP to cyclic AMP and can be used to estimate intact cell adenylate cyclase or cyclic nucleotide phosphodiesterase activities depending upon the specific protocol. Cyclic GMP accumulation was too low to be studied with intact cell prelabeling according to published procedures (Reynolds, P.E., S.J. Strada and W.J. Thompson, "Cyclic GMP accumulation in pulmonary microvascular endothelial cells measured by intact cell prelabeling," *Life Sci.,* 60:909-918, 1997).

### 2.C. Tissue sample assay

The PDE5 inhibitory activity of a test compound can also be determined from a tissue sample. Tissue samples, such as mammalian (preferably rat) liver, are collected from subjects exposed to the test compound. Briefly, a sample of tissue is homogenized in 500 µl of 6% TCA. A known amount of the homogenate is removed for protein analysis. The remaining homogenate is allowed to sit on ice for 20 minutes to allow for the protein to precipitate. Next, the homogenate is centrifuged for 30 minutes at 15,000g at 4°C. The supernatant is recovered and the pellet recovered. The supernatant is washed four times with five volumes of water saturated diethyl ether. The upper ether layer is discarded between each wash. The aqueous ether extract is dried in a speed vac. Once dried, the sample can be frozen for future use, or used immediately. The dried extract is dissolved in 500 µl of assay buffer. The amount of PDE5 inhibition is determined by assaying for the amount of cyclic nucleotides using an enzyme immunoassay (EIA), such as the Biotrak EIA system acetylation protocol (available from Amersham, Arlington Heights, IL, USA). Alternatively, RIA procedures as detailed above may be used.

### 2.D. Analyzing Results

The amount of inhibition is determined by comparing the activity of PDE5 in the presence and absence of the test compound. Inhibition of PDE5 activity is indicative that the compound is useful for treating neoplasia. Significant inhibitory activity greater than that of the benchmark, exisulind, preferably greater than 50% at a concentration of 10 µM or below, is indicative that a compound should be further evaluated for antineoplastic properties. Preferably, the IC₅₀ value for PDE5 inhibition should be less than 50 µM for the compound to be further considered for potential use.

### 3. Determining Whether A Compound Reduces The Number Of Tumor Cells

In an alternate embodiment, the screening method of the present invention involves further determining whether the compound reduces the growth of tumor cells. Various cell lines can be used in the sample depending on the tissue to be tested. For example, these cell lines include: SW-480 - colonic adenocarcinoma; HT-29 - colonic adenocarcinoma, A-427 - lung adenocarcinoma carcinoma; MCF-7 - breast adenocarcinoma; and UACC-375- melanoma line; and DU145 - prostrate carcinoma. Cytotoxicity data obtained using these cell lines are indicative of an inhibitory effect on neoplastic lesions. These cell lines are well characterized, and are used by the United States National Cancer Institute in their screening program for new anti-cancer drugs.

### 3A. Tumor Inhibition in HT-29 Cell Line

A compound's ability to inhibit tumor cell growth can be measured using the HT-29 human colon carcinoma cell line obtained from ATCC (Bethesda, MD). HT-29 cells have previously been characterized as relevant colon tumor cell culture model (Fogh, J., and Trempe, G. *In: Human Tumor Cells in Vitro,* J. Fogh (eds.), Plenum Press, New York, pp. 115-159, 1975). HT-29 cells are maintained in RPMI media supplemented with 5% fetal serum (Gemini Bioproducts, Inc., Carlsbad, CA) and 2 mm glutamine, and 1% antibiotic-antimycotic in a humidified atmosphere of 95% air and 5% CO₂ at 37°C. Briefly, HT-29 cells are plated at a density of 500 cells/well in 96 well microtiter plates and incubated for 24 hours at 37°C prior to the addition of compound. Each determination of cell number involved six replicates. After six days in culture, the cells are fixed by the addition of cold trichloroacetic acid to a final concentration of 10% and protein levels are measured using the sulforhodamine B (SRB) colorimetric protein stain assay as previously described by Skehan, P., Storeng, R., Scudiero, D., Monks, A., McMahon, J., Vistica, D., Warren, J.T., Bokesch, H., Kenney, S., and Boyd, M.R., "New Colorimetric Assay For Anticancer-Drug Screening,*" J*. *Natl*. *Cancer Inst.* 82: 1107-1112, 1990.

In addition to the SRB assay, a number of other methods are available to measure growth inhibition and could be substituted for the SRB assay. These methods include counting viable cells following trypan blue staining, labeling cells capable of DNA synthesis with BrdU or radiolabeled thymidine, neutral red staining of viable cells, or MTT staining of viable cells.

### 3.B. Analyzing Results

Significant tumor cell growth inhibition greater than about 50% at a dose of 100µM or below is further indicative that the compound is useful for treating neoplastic lesions. Preferably, an IC₅₀ value is determined and used for comparative purposes. This value is equivalent to the concentration of drug needed to inhibit tumor cell growth by 50% relative to the control. Preferably, the IC₅₀ value should be less than 100µM for the compound to be considered further for potential use for treating neoplastic lesions.

### 4. Determining Whether A Compound Induces Apoptosis

In a second alternate embodiment, the screening method of the present invention further involves determining whether the compound induces apoptosis in cultures of tumor cells.

Two distinct forms of cell death may be described by morphological and biochemical criteria: necrosis and apoptosis. Necrosis is accompanied by increased permeability of the plasma membrane; the cells swell and the plasma membrane ruptures within minutes. Apoptosis is characterized by membrane blebbing, condensation of cytoplasm and the activation of endogenous endonucleases.

Of the two, apoptosis is the most common form of eukaryotic cell death. It occurs naturally during normal tissue turnover and during embryonic development of organs and limbs. Apoptosis also is induced by cytotoxic T-lymphocytes and natural killer cells, by ionizing radiation and certain chemotherapeutic drugs. Inappropriate regulation of apoptosis is thought to play an important role in many pathological conditions including cancer, AIDS, Alzheimer disease, etc. Compounds can be screened for induction of apoptosis using cultures of tumor cells maintained under conditions as described above. Treatment of cells with test compounds involves either pre-or post-confluent cultures and treatment for two to seven days at various concentrations. Apoptotic cells are measured in both the attached and "floating" compartments of the cultures. Both compartments are collected by removing the supernatant, trypsinizing the attached cells, and combining both preparations following a centrifugation wash step (10 minutes, 2000 rpm). The protocol for treating tumor cell cultures with sulindac and related compounds to obtain a significant amount of apoptosis has been described in the literature. (See, Piazza, G.A., et al., *Cancer Research,* 55:3110-16, 1995). The novel features include collecting both floating and attached cells, identification of the optimal treatment times and dose range for observing apoptosis, and identification of optimal cell culture conditions.

### 4.A. Morphological observation of apoptosis

Following treatment with a test compound, cultures can be assayed for apoptosis and necrosis by florescent microscopy following labeling with acridine orange and ethidium bromide. The method for measuring apoptotic cell number has previously been described by Duke & Cohen, "Morphological And Biochemical Assays Of Apoptosis," *Current Protocols In Immunology,* Coligan et al., eds., 3.17.1-3.17.16 (1992).

For example, floating and attached cells can be collected by trypsinization and washed three times in PBS. Aliquots of cells can be centrifuged. The pellet can then be resuspended in media and a dye mixture containing acridine orange and ethidium bromide prepared in PBS and mixed gently. The mixture can then be placed on a microscope slide and examined.

### 4.B. Analysis of apoptosis by DNA fragmentation

Apoptosis can also be quantified by measuring an increase in DNA fragmentation in cells which have been treated with test compounds. Commercial photometric EIA for the quantitative *in vitro* determination of cytoplasmic histone-associated-DNA-fragments (mono- and oligonucleosomes) are available (Cell Death Detection ELISA^{okys}, Cat. No. 1,774,425, Boehringer Mannheim). The Boehringer Mannheim assay is based on a sandwich-enzyme-immunoassay principle using mouse monoclonal antibodies directed against DNA and histones, respectively. This allows the specific determination of mono- and oligonucleosomes in the cytoplasmatic fraction of cell lysates.

According to the vendor, apoptosis is measured in the following fashion. The sample (cell-lysate) is placed into a streptavidin-coated microtiter plate ("MTP"). Subsequently, a mixture of anti-histone-biotin and anti-DNA peroxidase conjugate are added and incubated for two hours. During the incubation period, the anti-histone antibody binds to the histone-component of the nucleosomes and simultaneously fixes the immunocomplex to the streptavidin-coated MTP via its biotinylation. Additionally, the anti-DNA peroxidase antibody reacts with the DNA component of the nucleosomes. After removal of unbound antibodies by a washing step, the amount of nucleosomes is quantified by the peroxidase retained in the immunocomplex. Peroxidase is determined photometrically with ABTS7 (2,2'-Azido-[3-ethylbenzthiazolin-sulfonate])∗ as substrate.

For example, SW-480 colon adenocarcinoma cells are plated in a 96-well MTP at a density of 10,000 cells per well. Cells are then treated with test compound, and allowed to incubate for 48 hours at 37°C. After the incubation, the MTP is centrifuged and the supernatant is removed. The cell pellet in each well is then resuspended in lysis buffer for 30 minutes. The lysates are then centrifuged and aliquots of the supernatant (i.e. cytoplasmic fraction) are transferred into streptavidin coated MTP. Care is taken not to shake the lysed pellets (i.e. cell nucleii containing high molecular weight, unfragmented DNA) in the MTP. Samples are then analyzed.

Fold stimulation (FS = ODₘₐₓ/ODᵥₑₕ), an indicator of apoptotic response, is determined for each compound tested at a given concentration. EC₅₀ values may also be determined by evaluating a series of concentrations of the test compound.

### 4.C. Analyzing Results

Statistically significant increases of apoptosis (i.e., greater than 2 fold stimulation at a concentration of 100µM are further indicative that the compound is useful for treating neoplastic lesions. Preferably, the EC₅₀ value for apoptotic activity should be less than 100µM for the compound to be further considered for potential use for treating neoplastic lesions. EC₅₀ is herein defined as the concentration that causes 50% induction of apoptosis relative to vehicle treatment.

### 5. VALIDATION - Mammary Gland Organ Culture Model Tests

Test compounds identified by the above methods can be tested for antineoplastic activity by their ability to inhibit the incidence of preneoplastic lesions in a mammary gland organ culture system. This mouse mammary gland organ culture technique has been successfully used by other investigators to study the effects of known antineoplastic agents such as NSAIDs, retinoids, tamoxifen, selenium, and certain natural products, and is useful for validation of the screening method of the present invention.

For example, female BALB/c mice can be treated with a combination of estradiol and progesterone daily, in order to prime the glands to be responsive to hormones *in vitro.* The animals are sacrificed and thoracic mammary glands are excised aseptically and incubated for ten days in growth media supplemented with insulin, prolactin, hydrocortisone, and aldosterone. DMBA (7,12-dimethylbenz(a)anthracene) is administered to induce the formation of premalignant lesions. Fully developed glands are then deprived of prolactin, hydrocortisone, and aldosterone, resulting in the regression of the glands but not the premalignant lesions.

The test compound is dissolved in DMSO and added to the culture media for the duration of the culture period. At the end of the culture period, the glands were fixed in 10% formalin, stained with alum carmine, and mounted on glass slides. The incidence of forming mammary lesions is the ratio of the glands with mammary lesions and glands without lesions. The incidence of mammary lesions in test compound treated glands is compared with that of the untreated glands.

The extent of the area occupied by the mammary lesions can be quantitated by projecting an image of the gland onto a digitation pad. The area covered by the gland is traced on the pad and considered as 100% of the area. The space covered by each of the unregressed structures is also outlined on the digitization pad and quantitated by the computer.

### EXPERIMENTAL SECTION

A number of test compounds were examined in the various protocols and screened for potential use in treating neoplasia. The results of these tests are reported below. The test compounds are hereinafter designated by a letter code that corresponds to the following:
A - rac-threo-(E)-1-(N,N'-diethylaminoethanethio)-1-(butan-1',4'-olido)-[3',4':1,2]-6-fluoro-2-methyl-3-(p-methylsulfonylbenzylidene)-indan;
B - (Z)-5-Fluoro-2-methyl-1-(3,4,5-trimethoxybenzylidene)-3-acetic acid;
C - (Z)-5-Fluoro-2-methyl-1-(p-chlorobenzylidene)-3-acetic acid;
D - rac-(E)-1-(butan-1',4'-olido)-[3',4':1,2]-6-fluoro-2-methyl-3-(p-methylsulfonylbenzylidene)-1S-indanyl-N-acetylcysteine;
E - (Z)-5-Fluoro-2-methyl-1-(3,4,5-trimethoxybenzylidene)-3-indenylacetamide, N-benzyl;
F - (Z)-5-Fluoro-2-methyl-1-(p-methylsulfonylbenzylidene)-3-indenylacetamide, N,N'-dicyclohexyl;
G - ribo-(E)-1-Triazolo-[2',3':1'',3'']-1-(butan-1',4'-olido)-[3',4': 1,2]-6-fluoro-2-methyl-3-(p-methylsulfonylbenzylidene)-indan; and
H - rac-(E)-1-(butan-1',4'-olido)-[3',4':1,2]-6-fluoro-2-methyl-3-(p-methylsulfonylbenzylidene)-1S-indanyl-glutathione).

### Example 1 - COX inhibition assay

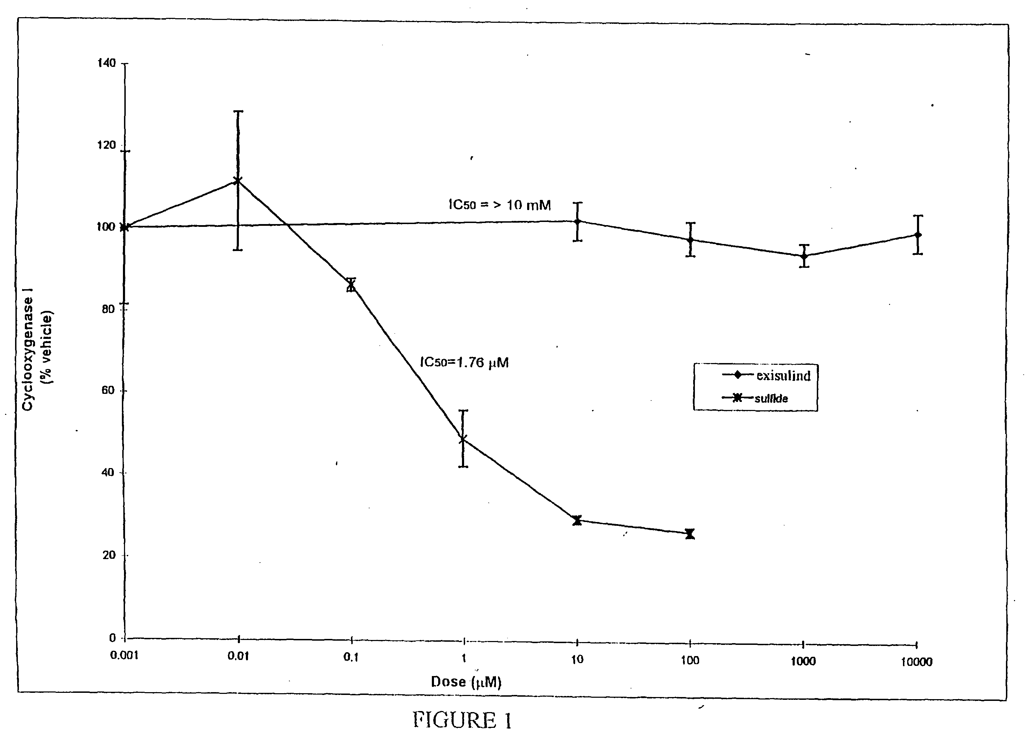

Reference compounds and test compounds were analyzed for their COX inhibitory activity in accordance with the protocol for the COX assay of section 1.B. *supra*. Figure 1 shows the effect of various concentrations of either sulindac sulfide or exisulind on purified cyclooxygenase (Type 1) activity. Cyclooxygenase activity was determined using purified cyclooxygenase from ram seminal vesicles as described previously (Mitchell et al, *supra*). The IC-50 value for sulindac sulfide was calculated to be approximately 1.76 µM, while that for exisulind was greater than 10,000 µM. These data show that sulindac sulfide, but not exisulind, is a COX-I inhibitor. Similar data was obtained for the COX-2 isoenzyme. (Thompson, et al., Journal of the National Cancer Institute, 87: 1259-1260, 1995).

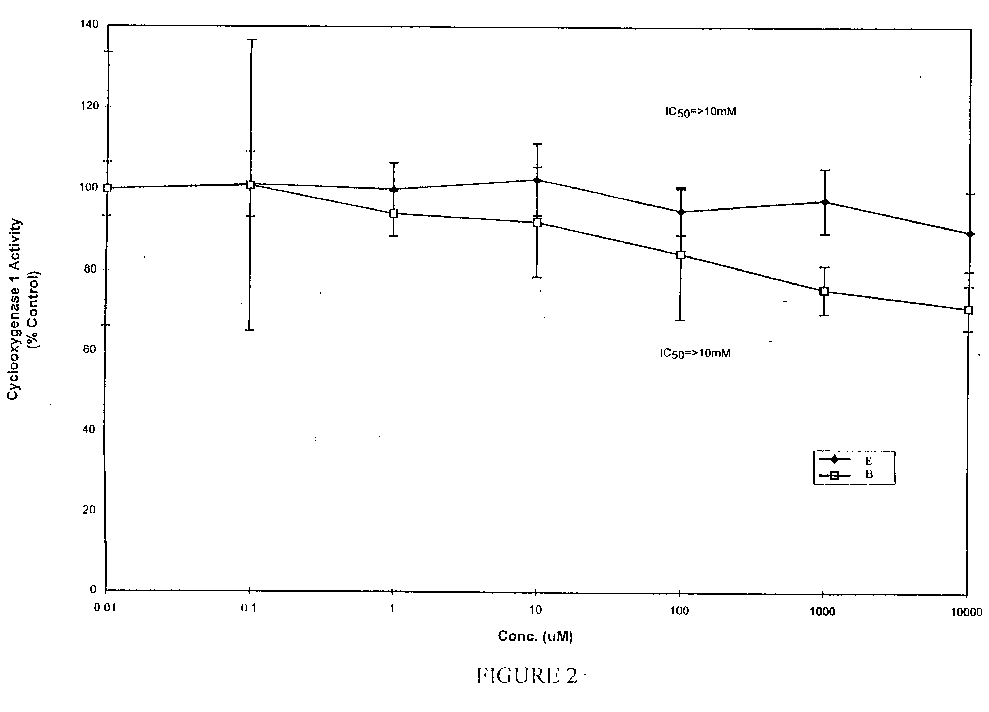

Figure 2 shows the effect of test compounds B and E on COX inhibition. COX activity was determined as for the compounds shown in Figure 1. The data show that both test compound B and E do not significantly inhibit COX-I.

**Table 1:**

| Cyclooxygenase inhibitory activity among a series of compounds | |
|---|---|
| Reference compounds | % Inhibition at 100 µM |
| Indomethacin | 95 |
| MY5445 | 94 |
| Sulindac sulfide | 97 |
| Exisulind | <25 |

| Test compounds | % Inhibition at 100 µM |
|---|---|
| A | <25 |
| B | <25 |
| C | 87 |
| D | <25 |
| E | <25 |

In accordance with the protocol of section 1.B., *supra,* compounds A through E were evaluated for COX inhibitory activity as reported in Table 1 above. Compound C was found to inhibit COX greater than 25% at a 100 µM dose, and therefore, would not be selected for further screening.

### Example 2 - PDE5 inhibition assay

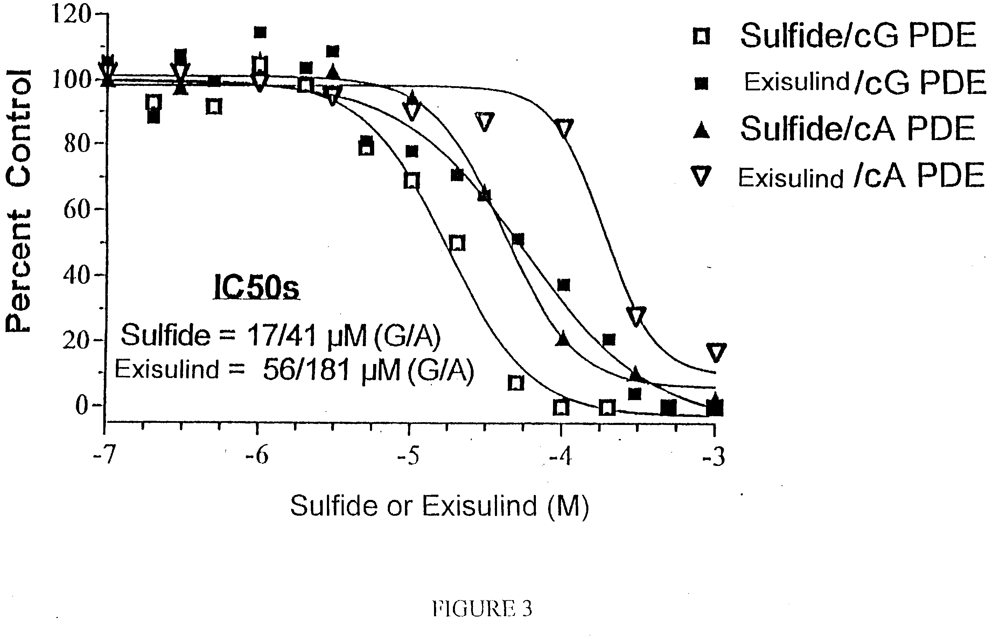

Reference compounds and test compounds were analyzed for their PDE5 inhibitory activity in accordance with the protocol for the assay of section 2.A., *supra.* Figure 3 shows the effect of various concentrations of sulindac sulfide and exisulind on either PDE-4 or PDE5 activity purified from human colon HT-29 cultured tumor cells, as described previously (W. J. Thompson et al., *supra).* The IC₅₀ value of sulindac sulfide for inhibition of PDE4 was 41 µM, and for inhibition of PDE5 was 17 µM. The IC₅₀ value of exisulind for inhibition of PDE4 was 181 µM, and for inhibition of PDE5 was 56 µM. These data show that both sulindac sulfide and exisulind inhibit phospohodiesterase activity. Both compounds show selectivity for the PDE5 isoenzyme form.

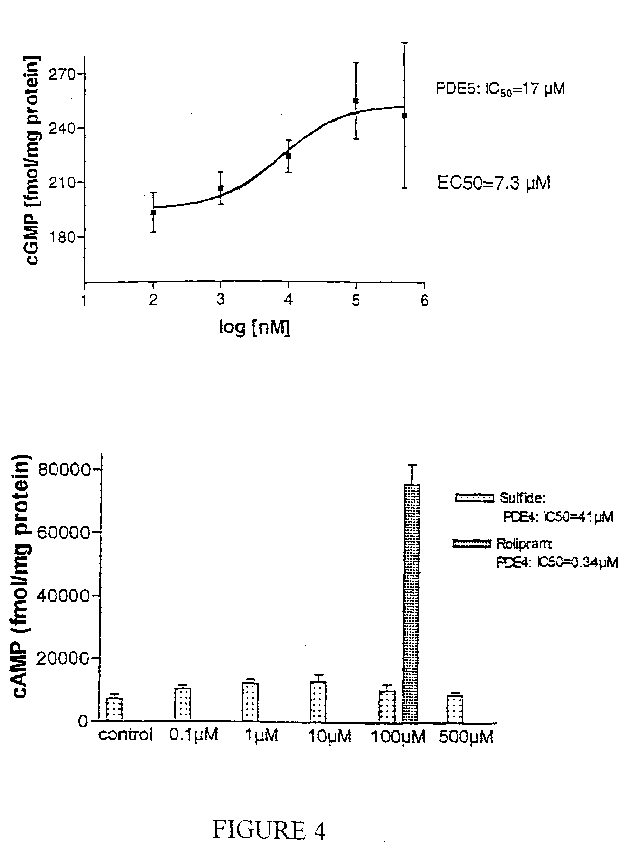

Figure 4 shows the effects of sulindac sulfide on either cGMP or cAMP production as determined on cultured HT-29 cells in accordance with the assay of section 2.B., *supra.* HT-29 cells were treated with sulindac sulfide for 30 minutes and cGMP or cAMP was measured by conventional radioimmunoassay method. As indicated, sulindac sulfide increased the levels of cGMP by greater than 50% with an EC₅₀ value of 7.3 µM (top). Levels of cAMP were unaffected by treatment, although a known PDE4 inhibitor, rolipram, increased cAMP (bottom). The data demonstrate the pharmacological significance of inhibiting PDE5, relative to PDE4.

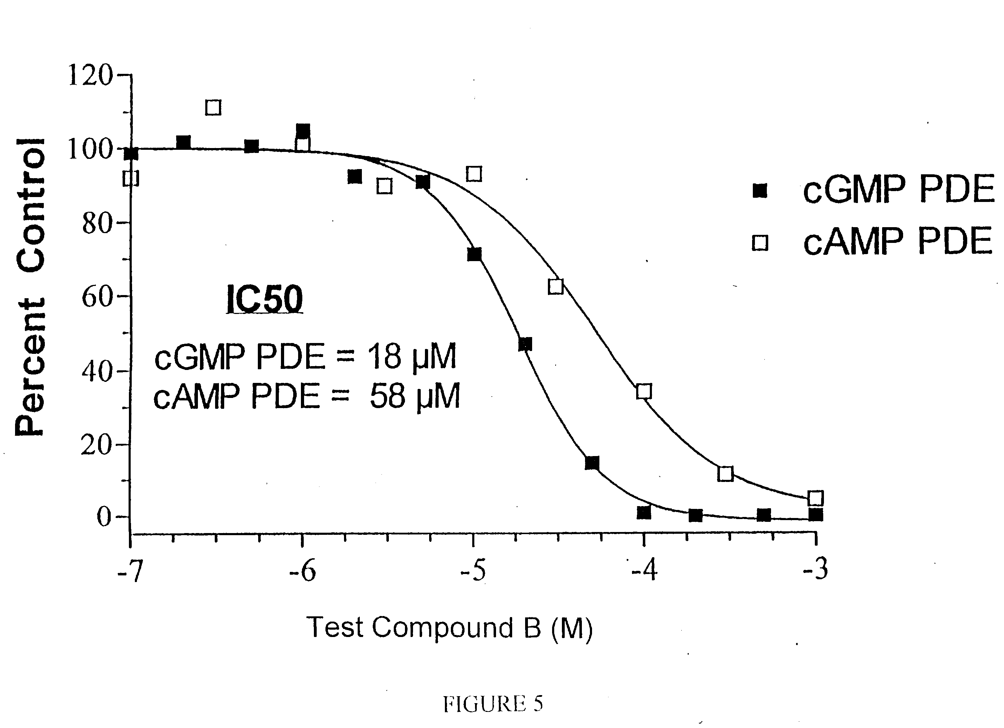

Figure 5 shows the effect of the indicated dose of test compound B on either PDE5 or PDE4 isozymes of phosphodiesterase. The calculated IC₅₀ value for PDE5 was 18 µM and 58 µM for PDE4.

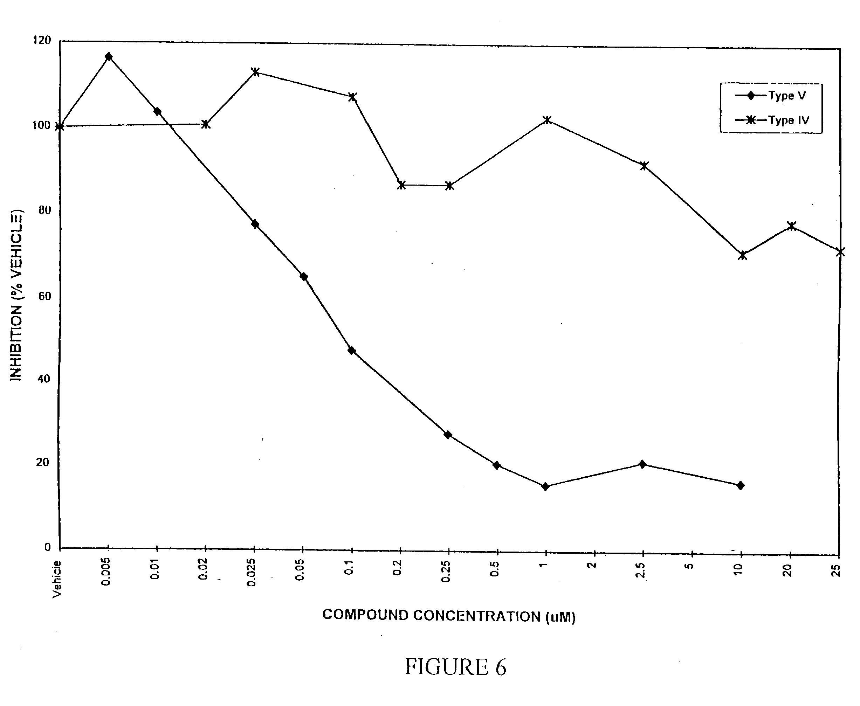

Figure 6 shows the effect of the indicated dose of test compound E on either PDE4 or PDE5. The calculated IC₅₀ value was 0.08 µM for PDE5 and greater than 25 µM for PDE4.

**Table 2:**

| PDE5 inhibitory activity among a series of compounds | |
|---|---|
| Reference compounds | % Inhibition at 10 µM |
| Indomethacin | 34 |
| MY5445 | 86 |
| Sulindac sulfide | 97 |
| Exisulind | 39 |

| Test compounds | % Inhibition at 10 µM |
|---|---|
| A | <25 |
| B | <25 |
| C | <25 |
| D | 36 |
| E | 75 |

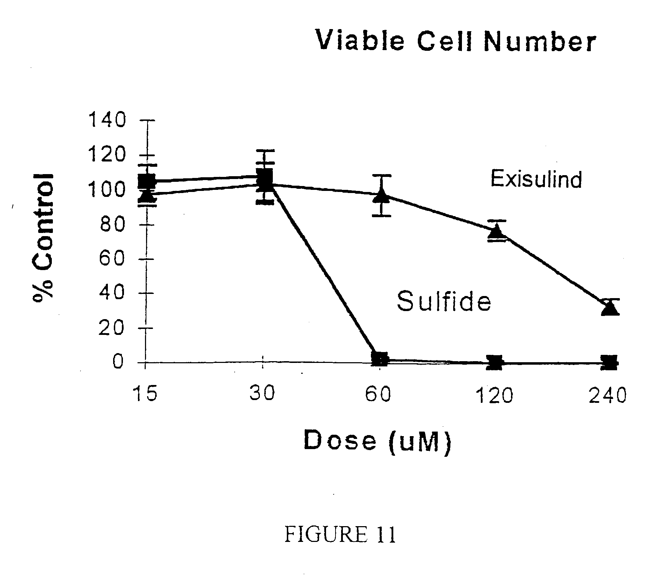

The above compounds in Table 2 were evaluated for PDE inhibitory activity, as described in the protocol of section 2.A; *supra.* Of the compounds that did not inhibit COX, only compound E was found to cause greater than 50% inhibition at 10 µM. As noted in Figure 11, compound B showed inhibition of greater than 50% at a dose of 20 µM. Therefore, depending on the dosage level used in a single dose test, some compounds may be screened out that otherwise may be active at slightly higher dosages. The dosage used is subjective and may be lowered after active compounds are found at certain levels to identify even more potent compounds.

### Example 3 - Apoptosis assay

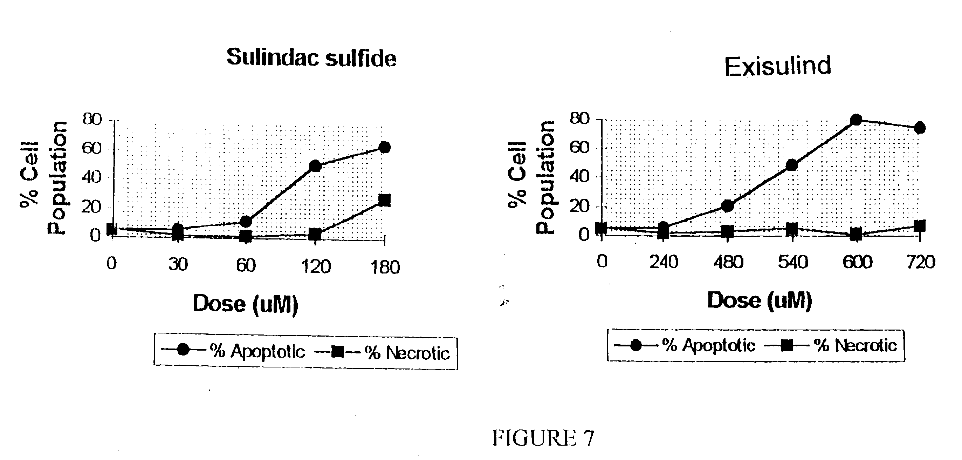

Reference compounds and test compounds were analyzed for their PDE5 inhibitory activity in accordance with the protocol for the assay of section 4.A. and 4.B., *supra.* In accordance with the assay of 4.A., Figure 7 shows the effects of sulindac sulfide and exisulind on apoptotic and necrotic cell death. HT-29 cells were treated for six days with the indicated dose of either sulindac sulfide or exisulind. Apoptotic and necrotic cell death was determined previously (Duke and Cohen, In: Current Protocols in Immunology, 3.17.1 - 3.17.16, New York, John Wiley and Sons, 1992). The data shows that both sulindac sulfide and exisulind are capable of causing apoptotic cell death without inducing necrosis. All data were collected from the same experiment.

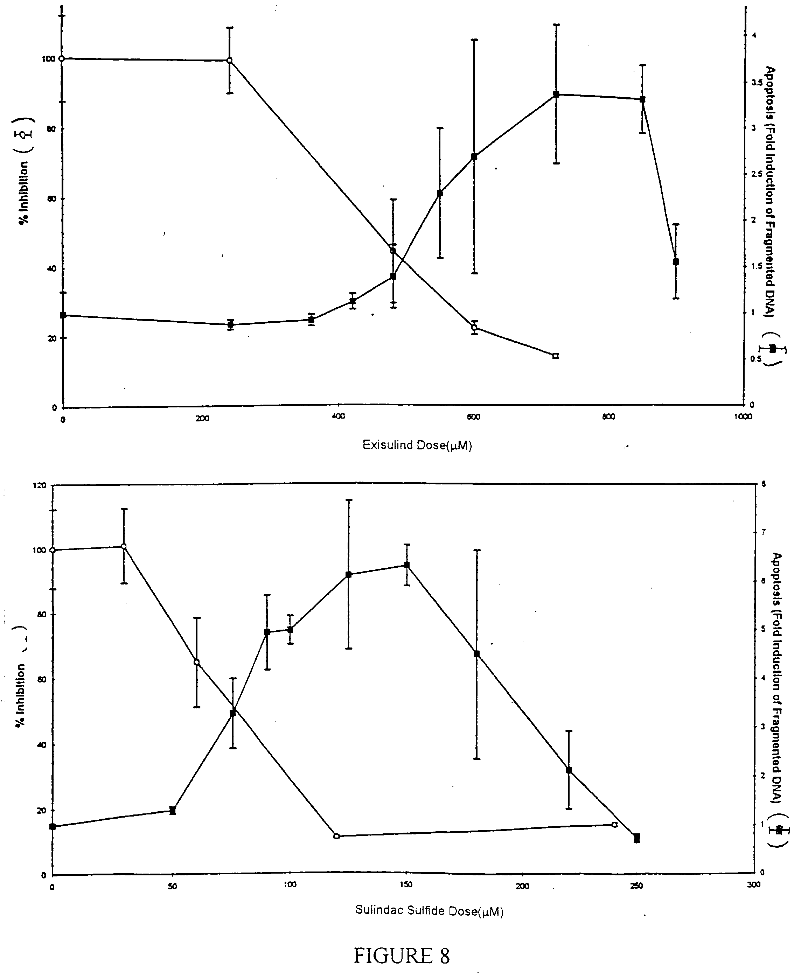

In accordance with the assay of 4.B., Figure 8 shows the effect of sulindac sulfide and sulfone on tumor growth inhibition and apoptosis induction as determined by DNA fragmentation. Top figure; growth inhibition (open symbols, right axis) and DNA fragmentation (closed symbols, left axis) by exisulind. Bottom figure; growth inhibition (open symbols) and DNA fragmentation (closed symbols) by sulindac sulfide. Growth inhibition was determined by the SRB assay after six days of treatment. DNA fragmentation was determined after 48 hours of treatment. All data was collected from the same experiment.

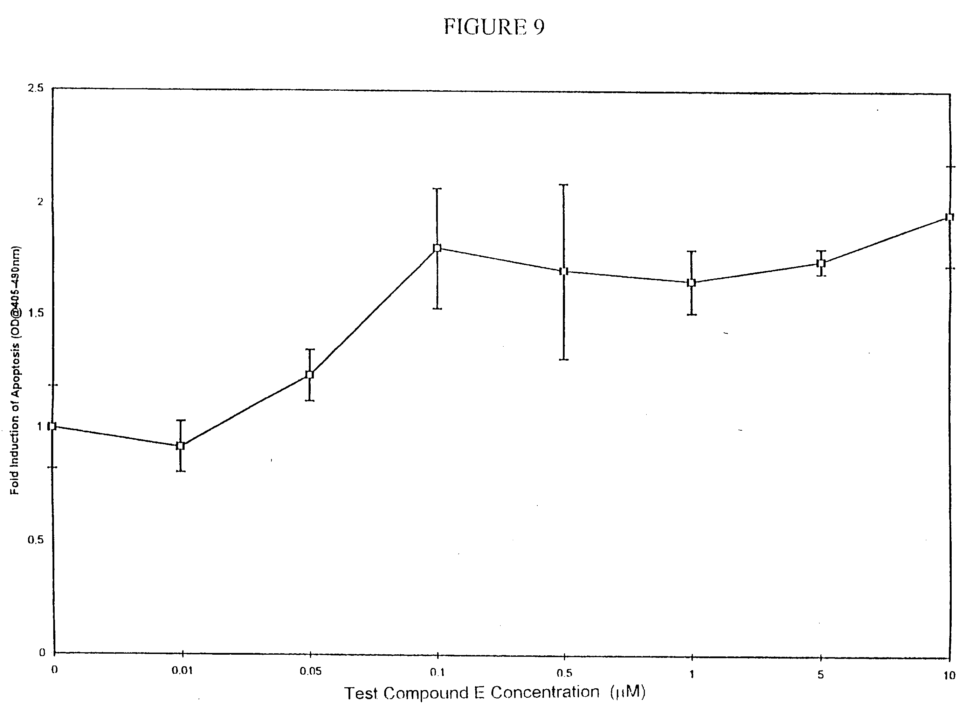

Figure 9 shows the apoptosis inducing properties of compound E. HT-29 colon adenocarcinoma cells were treated with the indicated concentration of compound E for 48 hours and apoptosis was determined by the DNA fragmentation assay. The calculated EC₅₀ value was 0.05 µM.

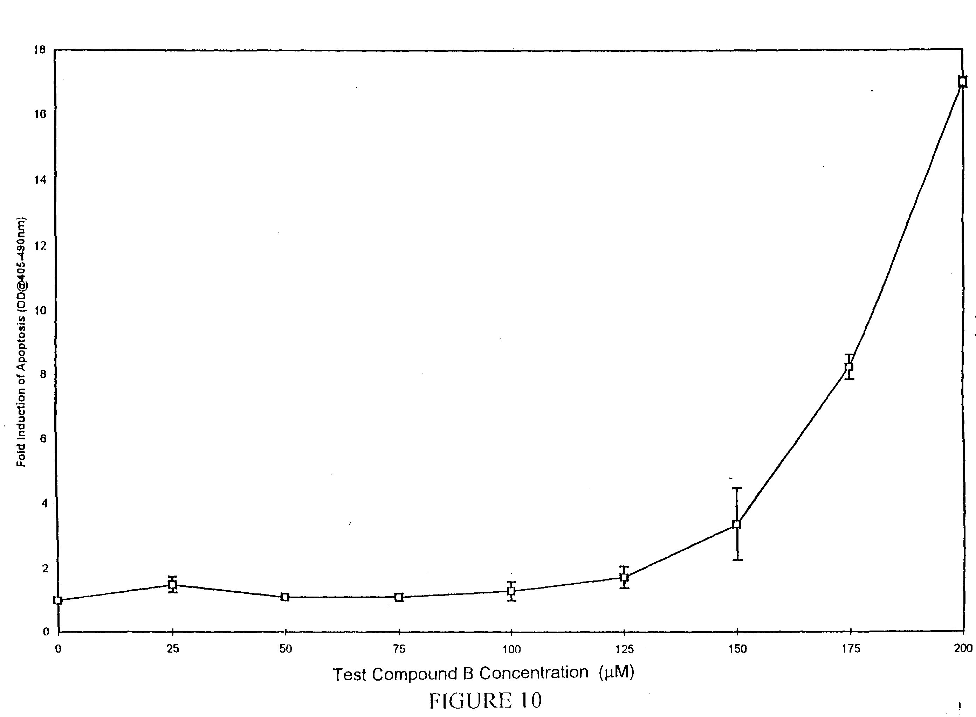

Figure 10 shows the apoptosis inducing properties of compound B. HT-29 colon adenocarcinoma cells were treated with the indicated concentration of compound B for 48 hours and apoptosis was determined by the DNA fragmentation assay. The calculated EC₅₀ value was approximately 175 µM.

**Table 3:**

| Apoptosis inducing activity among a series of compounds | |
|---|---|
| Reference compounds | Fold induction 100 µM |
| Indomethacin | <2.0 |
| MY5445 | 4.7 |
| Sulindac sulfide | 7.9 |
| Exisulind | <2.0 |

| Test compounds | Fold induction at 100 µM |
|---|---|
| A | <2.0 |
| B | 3.4 |
| C | 5.6 |
| D | <2.0 |
| E | 4.6 |

In accordance with the protocol of section 4.B., *supra,* the compounds A through E were tested for apoptosis inducing activity, as reported in Table 3 above. Compounds B, C and E showed significant apoptotic inducing activity, greater than 2.0 fold, at a dosage of 100 µM. Of these three compounds, at this dosage only B and E did not inhibit COX and inhibited PDE5.

The apoptosis inducing activity for a series of phosphodiesterase inhibitors was determined. The data are shown in Table 4 below. HT-29 cell were treated for 6 days with various inhibitors of phospohodiesterase. Apoptosis and necrosis were determined morphologically after acridine orange and ethidium bromide labelling in accordance with the assay of section 4.A., *supra.* The data show that PDE5 is useful for screening compounds that induce apoptosis of HT-29 cells.

**Table 4.**

| Apoptosis Inducing Data for PDE Inhibitors | | | |
|---|---|---|---|
| Inhibitor | Reported Selectivity | % Apoptosis | % Necrosis |
| Vehicle | | 8 | 6 |
| 8-methoxy-IBMX | PDE1 | 2 | 1 |
| Milrinone | PDE3 | 18 | 0 |
| RO-20-1724 | PDE4 | 11 | 2 |
| MY5445 | PDE5 | 80 | 5 |
| IBMX | Non-selective | 4 | 13 |

### Example 4 - Growth inhibition assay

Reference compounds and test compounds were analyzed for their PDE5 inhibitory activity in accordance with the protocol for the assay of section 3.A., *supra.* Figure 11 shows the inhibitory effect of various concentrations of sulindac sulfide and exisulind on the growth of HT-29 cells. HT-29 cells were treated for six days with various doses of exisulind (triangles) or sulfide (squares) as indicated. Cell number was measured by a sulforhodamine assay as previously described (Piazza et al., *Cancer Research,* 55: 3110-3116, 1995). The IC₅₀ value for the sulfide was approximately 45 µM and 200 µM for the sulfone. The data shows that both sulindac sulfide and exisulind are capable of inhibiting tumor cell growth.

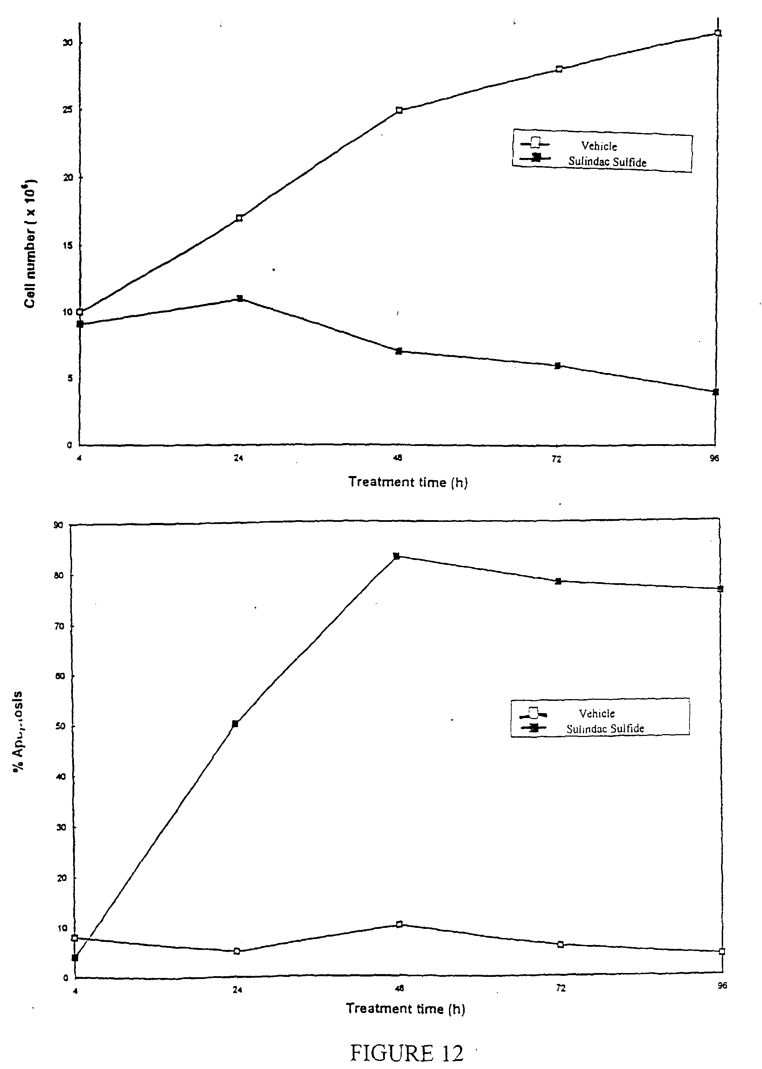

Figure 12 shows the growth inhibitory and apoptosis-inducing activity of sulindac sulfide. A time course experiment is shown involving HT-29 cells treated with either vehicle, 0.1% DMSO (open symbols) or sulindac sulfide, 120 µM (closed symbols). Growth inhibition (top) was measured by counting viable cells after trypan blue staining. Apoptosis (bottom) was measured by morphological determination following staining with acridine orange and ethidium bromide as described previously (Duke and Cohen, In: Current Protocols in Immunology, 3.17.1 - 3.17.16, New York, John Wiley and Sons, 1992). The data demonstrate that sulindac sulfide is capable of inhibiting tumor cell growth and that the effect is accompanied by an increase in apoptosis. All data were collected from the same experiment.

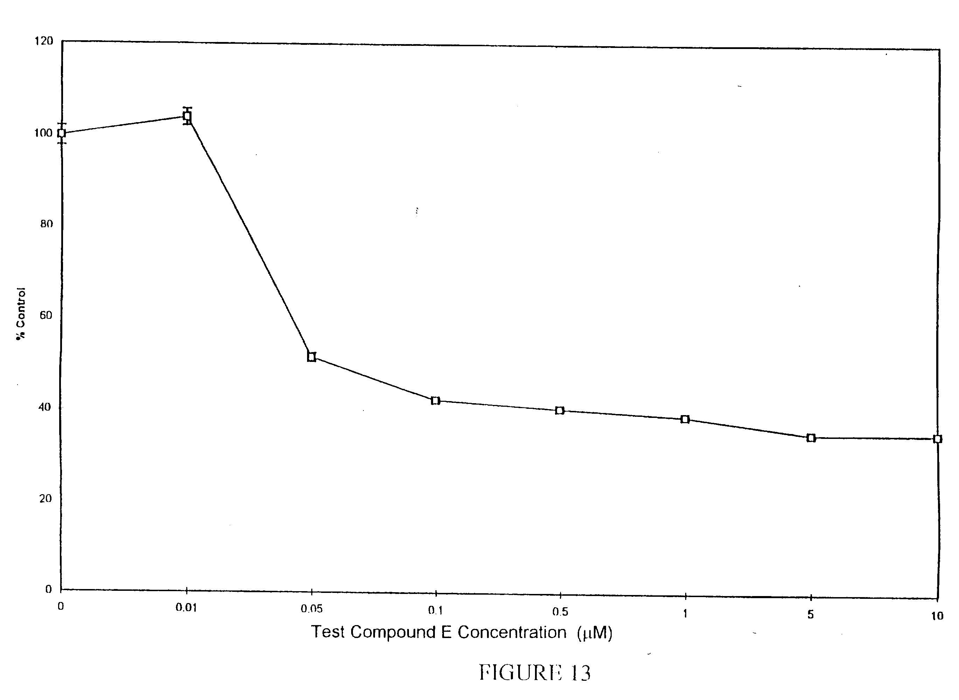

Figure 13 shows the growth inhibitory activity of test compound E. HT-29 colon adenocarcinoma cells were treated with the indicated concentration of comound E for six days and cell number was determined by the SRB assay. The calculated IC₅₀ value was 0.04 µM.

**Table 5:**

| Growth inhibitory activity among a series of compounds | |
|---|---|
| Reference compounds | % Inhibition at 100 µM |
| Indomethacin | 75 |
| MY5445 | 88 |
| Sulindac sulfide | 88 |
| Exisulind | <50 |

| Test compounds | % Inhibition at 100 µM |
|---|---|
| A | 68 |
| B | 77 |
| C | 80 |
| D | 78 |
| E | 62 |

In accordance with the screening protocol of section 3.A., *supra,* compounds A through E were tested for growth inhibitory activity, as reported in Table 5 above. All the test compounds showed activity exceeding the benchmark exisulind at a 100 µM single does test.

The growth inhibitory activity for a series of phosphodiesterase inhibitors was determined. The data are shown in Table 6 below. HT-29 cell were treated for 6 days with various inhibitors of phospohodiesterase. Cell growth was determined by the SRB assay in accordance with section 3.A., *supra.* The data show that inhibitors of PDE5 were effective for inhibiting tumor cell growth.

**Table 6.**

| Growth Inhibitory Data for PDE Inhibitors | | |
|---|---|---|
| Inhibitor | Reported Selectivity | Growth inhibition (IC₅₀, µM) |
| 8-methoxy-IBMX | PDE1 | >200 µM |
| Milrinone | PDE3 | >200 µM |
| RO-20-1724 | PDE4 | >200 µM |
| MY5445 | PDE5 | 5 µM |
| IBMX | Non-selective | >100 µM |

To show the effectiveness of this screening method on various forms of neoplasia, compounds were tested on numerous cell lines. The effects of sulindac sulfide and exisulind on various cell lines was determined. The data is shown in table 7 below. The IC₅₀ values were determined by the SRB assay. The data shows the broad effectiveness of these compounds on a broad range of neoplasia, with effectiveness at comparable dose range. Therefore, compounds identified by this invention should be useful for treating multiple forms of neoplasia.

**Table 7.**

| Growth inhibitory Data of Various Cell Lines | | |
|---|---|---|
| Cell Type/Tissue specificity | IC₅₀(µM) | |
| | Sulindac sulfide | Exisulind |
| HT-29, Colon | 60 | 120 |
| HCT116, Colon | 45 | 90 |
| MCF7/S, Breast | 30 | 90 |
| UACC375, Melanoma | 50 | 100 |
| A-427, Lung | 90 | 130 |
| Bronchial Epithelial Cells (normal) | 30 | 90 |
| NRK, Kidney (normal) | 50 | 180 |
| KNRK, Kidney (transformed) | 60 | 240 |
| Human Prostate Carcinoma PC3 | | 82 |

### Example 5 - Activity in mammary gland organ culture model

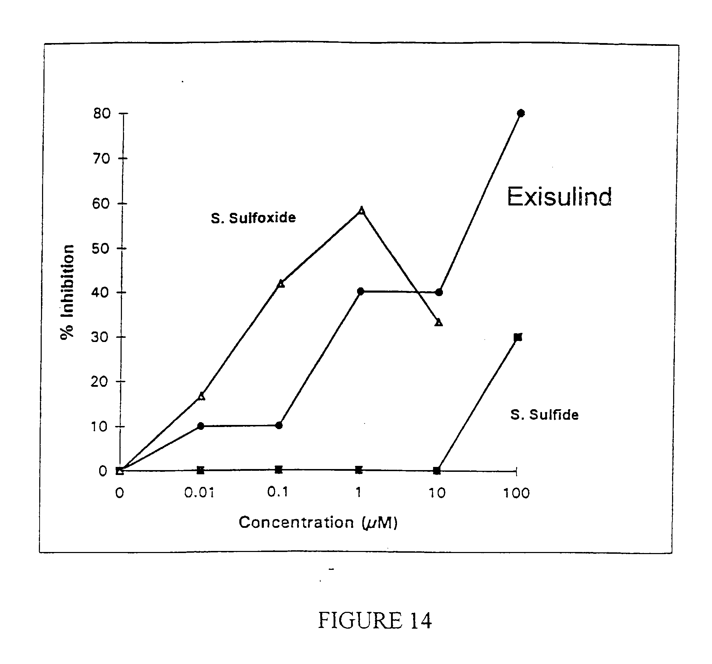

Figure 14 shows the inhibition of premalignant lesions in mammary gland organ culture by sulindac metabolites. Mammary gland organ culture experiment were performed as previously described (Mehta and Moon, *Cancer Research,* 46: 5832-5835, 1986). The results demonstrate that sulindac and exisulind effectively inhibit the formation of premalignant lesions, while sulindac sulfide was inactive. The data support the hypothesis that cyclooxygenase inhibition is not necessary for the anti-neoplastic properties of desired compounds.

### ANALYSIS

To identify compounds that have potential use for treating neoplasia, this invention provides a rationale for comparing experimental data of test compounds from several protocols. Within the framework of this invention, test compounds can be ranked according to their potential use for treating neoplasia in humans. Those compounds having desirable effects may be selected for more expensive and time consuming animal studies that are required to get approval before initiating human clinical trials.

Qualitative data of various test compounds and the several protocols are shown in Table 8 below. The data show that exisulind, compound B and compound E exhibit the appropriate activity to pass the screen of four assays: lack of COX inhibition, PDE inhibition, growth inhibition and apoptosis induction. The activity of these compounds in the mammary gland organ culture validates the effectiveness of this invention. The qualitative valuations of the screening protocols rank compound E best, then compound B and then exisulind.

**Table 8.**

| Activity Profile of Various Compounds | | | | | |
|---|---|---|---|---|---|
| Compound | COX Inhibition | PDE5 Inhibition | Growth Inhibition | Apoptosis | Mammary Gland Organ Culture |
| Exisulind | - | ++ | ++ | ++ | +++ |
| Sulindac sulfide | ++++ | +++ | +++ | +++ | - |
| MY5445 | ++++ | +++ | +++ | +++ | + |
| A | - | - | +++ | ++ | ++ |
| B | - | +++ | +++ | +++ | ++ |
| D | - | - | ++ | - | - |
| E | - | ++++ | ++++ | ++++ | ++++ |
| F | - | - | ++ | + | - |
| G | - | - | +++ | ++ | +++ |
| H | - | - | ++ | - | - |
| Table 8 Code: Activity of compounds based on evaluating a series of experiments involving tests for maximal | | | | | |
| activity and potency. | | | | | |
| - Not active | | | | | |
| + Slightly active | | | | | |
| + + Moderately active | | | | | |
| +++ Strongly active | | | | | |
| ++++ Highest activity ever recorded | | | | | |

Obviously, numerous modifications and variations of the present invention are possible in light of the above teachings. It is therefore to be understood that within the scope of the appended claims, the invention may be practiced otherwise than as specifically described herein.

## Claims

1. A method for identifying compounds with potential for treating neoplasia, comprising
determining the cyclooxygenase inhibitory activity of the compound; and
determining the cGMP-specific PDE inhibition activity of the compound by evaluating that activity against cGMP-specific PDE enzymatic activity;
wherein cyclooxygenase inhibitory activity lower than inhibitory activity of said cGMP-PDE activity is indicative that the compound has potential for treating neoplasia.

2. The method of claim 1 wherein said PDE is PDE5.

3. The method of claim 2, further comprising
determining whether the compound inhibits tumor cell growth in a culture;
wherein inhibition of tumor cell growth is further indicative that the compound has potential for treating neoplasia.

4. The method of claim 3 wherein the COX inhibitory activity of the compound is determined by:
contacting the compound with purified cyclooxygenase; and
measuring the change, if any, of cyclooxygenase activity;
wherein COX inhibitory activity of less than 25% at a concentration of about 100µM is indicative that the compound should be evaluated further for potential for treating neoplasia.

5. The method of claim 3, wherein the COX inhibitory activity of the compound is determined by:
contacting the compound with a cell which secretes PGE-2; and measuring the decrease, if any, of the PGE-2 secretion from the cell;
wherein a decrease in PGE-2 secretion correlates to a decrease in prostaglandin synthetase activity.

6. The method of claim 3, further comprising
determining whether the compound induces apoptosis of a tumor cell;
wherein induction of apoptosis is further indicative that the compound has potential for treating neoplasia.

7. The method of claim 6, further comprising
determining whether the compound inhibits tumor cell growth in a sample;
wherein inhibition of tumor cell growth is further indicative that the compound is useful for treating neoplasia.

8. The method of claim 7, wherein PDE-5 activity is determined by:
contacting the compound with a cell culture; and
determining the intracellular cyclic GMP concentration;
wherein PDE-inhibitory activity greater than 50% at a concentration of 10 µM is indicative that a compound should be evaluated further for potential for treating neoplasia.

9. The method of claim 7, wherein PDE-5 activity is determined by:
determining the ratio of intracellular cyclic GMP/cyclic AMP concentration;
wherein a ratio increase greater than three-fold at a concentration of 10 µM is indicative that a compound should be evaluated further for potential for treating neoplasia.

10. The method of claim 7, wherein apoptosis is determined by:
contacting the compound with a cell culture; and
determining if the compound increased the amount of fragmented DNA in the cytoplasm;
wherein increases of apoptosis of greater than 2 fold stimulation at a concentration of 100 µM are further indicative that the compound should be evaluated further for potential for treating neoplasia.

11. A method of selecting a compound for treating neoplasia, comprising determining the neoplastic cell growth inhibitory activity of the compound;
determining the cGMP-specific PDE inhibition activity of the compound; and
selecting the compound that exhibit growth inhibitory activity and said cGMP-specific enzyme inhibitory activity.

12. The method of claim 11 wherein said PDE is PDE5.

13. The method of claim 11, further comprising
selecting the compound where the IC₅₀ value for growth inhibitory activity is less than about 100 µM for use in treating neoplasia.

14. The method of claim 11, further comprising
determining whether the compound induces apoptosis in a cell; and
selecting the compound that induces apoptosis.

15. The method of claim 13, further comprising
selecting the compound where the EC₅₀ value for apoptotic activity is less than about 100 µM.

16. The method of claim 12, further comprising:
determining the COX inhibitory activity of the compound; and
selecting the compound with COX inhibitory activity lower than PDE-5 inhibitory activity.

17. A method for identifying a compound with potential for treating neoplasia, comprising:
selecting a compound with cGMP-specific PDE inhibiting activity; and
evaluating the neoplastic cell growth inhibiting activity of the compound wherein a compound that has said cGMP enzyme inhibiting activity and neoplastic cell growth inhibiting activity has the potential to inhibit neoplasia without substantially inhibiting the growth of normal cells.

18. A method for identifying a compound with potential for treating neoplasia, comprising:
treating neoplastic cells with the compound to be evaluated;
determining the intracellular amount of cGMP in the treated cells;
determining the intracellular amount of cAMP in the treated cells;
wherein an increase in the ratio of cGMP/cAMP in the treated cells compared to the ratio of cGMP/cAMP in untreated neoplastic cells is indicative that the compound has potential for treating neoplasia.

## Patentansprüche

1. Verfahren zur Identifizierung von zur Behandlung von Neoplasie geeigneten Verbindungen mit folgenden Verfahrensschritten:
- Bestimmung der Cyclooxygenase (COX) hemmenden Wirkung der Verbindung; und
- Bestimmung der cGMP-spezifischen PDE-Hemmungswirkung der Verbindung durch Ermittlung der Wirkung gegenüber der cGMP-spezifischen PDE-Enzym-Aktivität;
- wobei eine Eignung der Verbindung zur Behandlung von Neoplasie angezeigt wird, wenn die Cyclooxygenase hemmende Wirkung niedriger ist als die Hemmungswirkung der cGMP-spezifischen PDE-Aktivität

2. Verfahren nach Anspruch 1, dadurch gekennzeichnet, dass die PDE vom Typ PDE5 ist.

3. Verfahren nach Anspruch 2, ferner gekennzeichnet durch den Verfahrenschritt
- Untersuchung, ob die Verbindung das Wachstum von Tumorzellen in einer Zellkultur hemmt;
- wobei eine Eignung der Verbindung zur Behandlung von Neoplasie angezeigt wird, wenn das Tumorzellenwachstum gehemmt wird.

4. Verfahren nach Anspruch 3, dadurch gekennzeichnet, dass die COX hemmende Aktivität der Verbindung auf folgende Weise bestimmt wird:
- Man bringt die Verbindung mit gereinigter Cyclooxygenase in Kontakt; und
- mißt die Veränderung der Wirksamkeit der Cyclooxygenase, falls eine solche feststellbar ist;
- wobei eine die COX hemmende Wirkung von weniger als 25 % bei einer Konzentration von etwa 100 µM anzeigt, dass die Verbindung als geeignet zur Behandlung von Neoplasie eingestuft werden sollte.

5. Verfahren nach Anspruch 3, dadurch gekennzeichnet, dass die COX hemmende Aktivität der Verbindung auf folgende Weise bestimmt wird:
- Man bringt die Verbindung mit einer Zelle in Kontakt, welche PGE-2 ausschüttet; und
- mißt den Rückgang der PGE-2-Abscheidung der Zelle, falls ein solcher feststellbar ist;
- wobei einem Rückgang der PGE-2-Abscheidung auch ein Rückgang der Prostaglandin-Synthesewirkung entspricht.

6. Verfahren nach Anspruch 3, ferner gekennzeichnet durch den Verfahrenschritt
- Untersuchung, ob die Verbindung bei einer Tumorzelle Apoptosis auslöst;
- wobei die Auslösung von Apoptosis weiterhin die Eignung der Verbindung zur Behandlung von Neoplasie anzeigt.

7. Verfahren nach Anspruch 6, ferner gekennzeichnet durch den Verfahrenschritt
- Untersuchung, ob die Verbindung das Wachstum von Tumorzellen in einer Probe hemmt;
- wobei eine Hemmung des Wachstums von Tumorzellen weiterhin die Eignung der Verbindung zur Behandlung von Neoplasie anzeigt.

8. Verfahren nach Anspruch 7, dadurch gekennzeichnet, dass die PDE5-Aktivität auf folgende Weise bestimmt wird:
- Die Verbindung wird mit einer Zellkultur in Kontakt gebracht; und
- Bestimmung der intrazellulären Konzentration von cyclischem GMP;
- wobei eine PDE-Hemmungs-Wirkung von mehr als 50% bei einer Konzentration von 10 µM anzeigt, dass die Verbindung als geeignet für die Behandlung von Neoplasie eingestuft werden sollte.

9. Verfahren nach Anspruch 7, dadurch gekennzeichnet, dass die PDE5-Aktivität auf folgende Weise bestimmt wird:
- Bestimmung des Konzentrationsverhältnisses von intrazellulärem cyclischem GMP zu cyclischem AMP;
- wobei eine Erhöhung dieses Verhältnisses auf mehr als das Dreifache bei einer Konzentration von 10 µM anzeigt, dass die Verbindung als geeignet für die Behandlung von Neoplasie eingestuft werden sollte.

10. Verfahren nach Anspruch 7, dadurch gekennzeichnet, dass die Apoptosis auf folgende Weise untersucht wird:
- Man bringt die Verbindung in Kontakt mit einer Zellkultur; und
- Untersucht, ob die Verbindung die Konzentration an fragmentierter DNS in dem Zytoplasma erhöht;
- wobei ein Anstieg der Apoptosis auf mehr als die zweifache Stimulation bei einer Konzentration von 100 µM anzeigt, dass die Verbindung als geeignet für die Behandlung von Neoplasie eingestuft werden sollte.

11. Verfahren zur Auswahl einer Verbindung für die Behandlung von Neoplasie, umfassend die folgenden Verfahrensschritte:
- Untersuchung der wachstumshemmenden Wirkung der Verbindung, auf neoplastische Zellen;
- Untersuchung der cGMP-spezifischen, PDE5 hemmenden Wirkung der Verbindung; und
- Auswahl derjenigen Verbindung, welche sowohl eine wachstumshemmende Wirkung als auch eine cGMP-spezifische, enzymhemmende Wirkung aufweist.

12. Verfahren nach Anspruch 11, dadurch gekennzeichnet, dass die PDE vom Typ PDE5 ist.

13. Verfahren nach Anspruch 11, ferner gekennzeichnet durch den Verfahrenschritt:
- Auswahl derjenigen Verbindung zur Behandlung von Neoplasie, bei welcher der IC₅₀-Wert für die wachstumshemmende Wirkung weniger als etwa 100 µM beträgt.

14. Verfahren nach Anspruch 11, ferner gekennzeichnet durch den Verfahrenschritt:
- Untersuchung, ob die Verbindung in einer Zelle Apoptosis auslöst; und
- Auswahl derjenigen Verbindung, welche Apoptosis auslöst.

15. Verfahren nach Anspruch 13, ferner gekennzeichnet durch den Verfahrenschritt:
- Auswahl derjenigen Verbindung, bei welcher der EC₅₀-Wert für die Apoptosis-Wirkung weniger als etwa 100 µM beträgt.

16. Verfahren nach Anspruch 12, ferner gekennzeichnet durch die Verfahrenschritte:
- Untersuchung der COX hemmenden Wirkung der Verbindung; und
- Auswahl derjenigen Verbindung, deren COX hemmende Wirkung niedriger ist als ihre PDE-5 hemmende Wirkung.

17. Verfahren zur Identifizierung einer zur Behandlung von Neoplasie geeigneten Verbindung mit folgenden Verfahrensschritten:
- Auswahl einer Verbindung mit cGMP-spezifischer, PDE hemmenden Wirkung; und
- Untersuchung der der Verbindung auf ihre wachstumshemmende Wirkung auf neoplastische Zellen;
- wobei sich eine Verbindung, welche sowohl die cGMP-spezifische Enzym-Hemmungswirkung als auch die wachstumshemmende Wirkung auf neoplastische Zellen zeigt, zur Hemmung von Neoplasmen eignet, ohne das Wachstum normaler Zellen merklich zu beeinträchtigen.

18. Verfahren zur Identifizierung einer zur Behandlung von Neoplasie geeigneten Verbindung mit folgenden Verfahrensschritten:
- Behandlung neoplastischer Zellen mit der zu bewertenden Verbindung;
- Untersuchung der intrazellulären Menge von cGMP in den behandelten Zellen;
- Untersuchung der intrazellulären Menge von cAMP in den behandelten Zellen;
- wobei eine Erhöhung des Verhältnisses von cGMP zu cAMP in den behandelten .Zellen gegenüber dem Verhältnis von cGMP zu cAMP in den unbehandelten neoplastischen Zellen die Eignung der Verbindung für die Behandlung von Neoplasie anzeigt.

## Revendications

1. Méthode pour identifier des composés présentant un potentiel de traitement d'une néoplasie, comprenant
la détermination de l'activité inhibitrice de cycle-oxygénase du composé ; et
la détermination de l'activité inhibitrice de PDE spécifique de cGMP du composé en évaluant cette activité par rapport à une activité enzymatique de PDE spécifique de cGMP ;
dans laquelle une activité inhibitrice de cyclo-oxygénase inférieure à l'activité inhibitrice de ladite activité de cGMP-PDE est une indication que le composé présente un potentiel de traitement d'une néoplasie.

2. Méthode suivant la revendication 1, dans laquelle la PDE consiste en PDE5.

3. Méthode suivant la revendication 2, comprenant en outre
l'étape consistant à déterminer si le composé inhibe la croissance de cellules tumorales dans une culture ;
dans laquelle l'inhibition de la croissance de cellules tumorales est une indication supplémentaire que le composé présente un potentiel de traitement d'une néoplasie.

4. Méthode suivant la revendication 3, dans laquelle l'activité inhibitrice de COX du composé est déterminée :
en mettant en contact le composé avec de la cyclo-oxygénase purifiée ; et
en mesurant la variation, s'il en existe une quelconque, de l'activité de cyclo-oxygénase,
dans laquelle une activité inhibitrice de COX inférieure à 25 % à une concentration d'environ 100 µM est une indication que le composé doit être soumis à une évaluation supplémentaire pour le potentiel de traitement d'une néoplasie.

5. Méthode suivant la revendication 3, dans laquelle l'activité inhibitrice de COX du composé est déterminée :
en mettant en contact le composé avec une cellule qui sécrète de la PGE-2 ; et en mesurant la diminution, s'il en existe une quelconque, de la sécrétion de PGE-2 par la cellule ;
dans laquelle une diminution de la sécrétion de PGE-2 présente une corrélation avec une diminution de l'activité de prostaglandine-synthétase.

6. Méthode suivant la revendication 3, comprenant en outre
l'étape consistant à déterminer si le composé induit une apoptose d'une cellule tumorale;
dans laquelle l'induction d'une apoptose constitue une indication supplémentaire que le composé présente un potentiel de traitement d'une néoplasie.

7. Méthode suivant la revendication 6, comprenant en outre
l'étape consistant à déterminer si le composé inhibe la croissance de cellules tumorales dans un échantillon ;
dans laquelle l'inhibition de la croissance de cellules tumorales est une indication supplémentaire que le composé est utile pour le traitement d'une néoplasie.

8. Méthode suivant la revendication 7, dans laquelle l'activité de PDE-5 est déterminée :
en mettant en contact le composé avec une culture cellulaire ; et
en déterminant la concentration de GMP cyclique intracellulaire ;
dans laquelle une activité inhibitrice de PDE supérieure à 50 % à une concentration de 10 µM est une indication qu'un composé doit être soumis à une évaluation supplémentaire pour le potentiel de traitement d'une néoplasie.

9. Méthode suivant la revendication 7, dans laquelle l'activité de PDE-5 est déterminée :
en déterminant le rapport des concentrations intracellulaires de GMP cyclique/AMP cyclique ;
dans laquelle une augmentation du rapport supérieure à un facteur trois à une concentration de 10 µM est une indication qu'un composé doit être soumis à une évaluation supplémentaire pour le potentiel de traitement d'une néoplasie.

10. Méthode suivant la revendication 7, dans laquelle l'apoptose est déterminée :
en mettant en contact le composé avec une culture cellulaire ; et
en déterminant si le composé a augmenté la quantité d'ADN fragmenté dans le cytoplasme ;
dans laquelle des augmentations d'apoptose supérieures à une stimulation d'un facteur 2 à une concentration de 100 µM constituent une indication supplémentaire que le composé doit être soumis à une évaluation supplémentaire pour le potentiel de traitement d'une néoplasie.

11. Méthode de sélection d'un composé pour le traitement d'une néoplasie, comprenant la déterminaton de l'activité inhibitrice de croissance de cellules néoplasiques du composé ;
la détermination de l'activité inhibitrice de PDE spécifique de cGMP du composé ; et
la sélection du composé qui présente une activité inhibitrice de croissance et ladite activité inhibitrice d'enzyme spécifique du cGMP.

12. Méthode suivant la revendication 11, dans laquelle la PDE consiste en PDE5.

13. Méthode suivant la revendication 11, comprenant en outre
la sélection du composé pour lequel la valeur de CI₅₀ d'activité inhibitrice de croissance est inférieure à environ 100 µM à des fins d'utilisation dans le traitement d'une néoplasie.

14. Méthode suivant la revendication 11, comprenant en outre
l'étape consistant à déterminer si le composé induit une apoptose dans une cellule ; et
la sélection du composé qui induit une apoptose.

15. Méthode suivant la revendication 13, comprenant en outre,
la sélection du composé pour lequel la valeur de EC₅₀ d'activité apoptosique est inférieure à environ 100 µM.

16. Méthode suivant la revendication 12, comprenant en outre :
la détermination de l'activité inhibitrice de COX du composé ; et
la sélection du composé ayant une activité inhibitrice de COX inférieure à l'activité inhibitrice de PDE-5.

17. Méthode pour identifier un composé présentant un potentiel de traitement d'une néoplasie, comprenant :
la sélection d'un composé ayant une activité inhibitrice de PDE spécifique de cGMP ; et
l'évaluation de l'activité inhibitrice de croissance de cellules néoplasiques du composé, dans laquelle un composé qui présente ladite activité inhibitrice d'enzyme spécifique de cGMP et ladite activité inhibitrice de croissance de cellules néoplasiques présente le potentiel d'inhiber une néoplasie sans inhiber notablement la croissance des cellules normales.

18. Méthode pour identifier un composé présentant un potentiel de traitement d'une néoplasie, comprenant :
le traitement de cellules néoplasiques avec le composé à évaluer ;
la détermination de la quantité intracellulaire de cGGMP dans les cellules traitées ;
la détermination de la quantité intracellulaire de cAMP dans les cellules traitées ;
dans laquelle une augmentation du rapport cGMP/cAMP dans les cellules traitées comparativement au rapport cGMP/cAMP dans les cellules néoplasiques non traitées est une indication que le composé présente un potentiel de traitement d'une néoplasie.
